# EUROPEAN PATENT SPECIFICATION

(11) **EP 4 498 924 B1**
(45) Date of publication and mention of the grant of the patent: **28.10.2026**
(21) Application number: 23712552.1
(22) Date of filing: 22.03.2023
(51) Int. Cl.: A61B 8/06, A61B 8/00, A61B 8/08

(54) **FLOW MEASUREMENT**
FLUSSMESSUNG
MESURE DE DÉBIT

(30) Priority: 30.03.2022 EP 22165392
(43) Date of publication of application: 05.02.2025
(73) Proprietor: Koninklijke Philips N.V., 5656 AE Eindhoven (NL)
(72) Inventor: DIRKSEN, Peter, 5656 AG Eindhoven (NL); HAARTSEN, Jaap Roger, 5656 AG Eindhoven (NL); KLOOTWIJK, Johan Hendrik, 5656 AG Eindhoven (NL); VAN RENS, Antonia Cornelia, 5656 AG Eindhoven (NL)
(74) Representative: Philips Intellectual Property & Standards
(86) International application number: PCT/EP2023/057299
(87) International publication number: WO 2023/186651

(56) References cited:
- WO-A1-88/01850
- US-A- 5 394 876
- US-A1- 2021 160 621

## Description

### FIELD OF THE INVENTION

The present invention relates to flow measurement, and relates in particular to a flow measurement device, to an ultrasound imaging system for flow measurement and to a driving circuit for operating a sensor for flow measurement.

### BACKGROUND OF THE INVENTION

To measure blood flow in a vessel, devices such as flow wires are used, comprising a miniature piezoelectric transducer at the distal end of an elongate body. Based on the Doppler ultrasound principle, the flow wire provides blood flow velocity based on intravascular measurements. The measured Doppler frequency shift is converted into the blood velocity distribution in the arteries. The miniature piezoelectric element is connected by a long wire to the console. However, it has been shown that effects like a low signal to noise ratio (SNR), a poor bandwidth and resonance frequency variability may result in unsatisfactory measurement quality. There is a need to provide flow measurement with improved accuracy.

WO 1988/01850 A1, according to its abstract, relates to a non-invasive ultrasonic volume blood measurement system that includes a transducer probe assembly having an inner and a concentric outer element. Both are used to insonify a lumen area and to generate received signals representative of wide and narrow beams. The signals are processed simultaneously to produce respective Doppler power spectrums. Phase quadrature signals are generated for determining flow direction, and an AGC circuit provides output signals controlling the amplitude of both spectrums for accurate computation of beam power ratio. A mean frequency estimator circuit determines flow velocity at the lumen area, and a recording thereof is observed by the operator to ensure correct placement of the probe assembly for insonifying a desired lumen area. Thereafter, a processing/controlling unit determines volume blood flow by multiplying the determined flow velocity by the ratio of the obtained powers in the wide and narrow beams.

US 5394876 A1, according to its abstract, relates to aiming a pulse Doppler flow sensing device. The Doppler flow device transmits ultrasonic energy in a wide beam which uniformly encompasses a blood vessel and receives the reflected ultrasonic energy in the same wide beam and also in a beam which is small enough to be placed within the blood vessel. When the beams are concentric with the blood vessel, the device is correctly placed. A processor/controller process the received reflected Doppler energy and calculates the maximum and mean blood velocities from both beams. It also tests whether the blood velocities satisfy predetermined criteria. If the criteria are satisfied, the Doppler device is properly aligned with the blood vessel and the measurement is valid

US 2021/160621 A1, according to its abstract, relates to ultrasound circuits that employ a differential ultrasonic transducer element, such as a differential micromachined ultrasonic transducer element. The differential ultrasonic transducer element may be coupled to an integrated circuit that is configured to operate the differential ultrasonic transducer element in one or more modes of operation, such as a differential receive mode, a differential transmit mode, a single-ended receive mode, and a single-ended transmit mode Further relevant prior art systems and methods are disclosed in the patent publications US 2013/128702 A1 and JP S63 154167 A.

### SUMMARY OF THE INVENTION

The object of the present invention is solved by the subject-matter of the independent claim; further embodiments are incorporated in the dependent claims. It should be noted that the following described aspects of the invention apply also for the flow measurement device, for the ultrasound imaging system for flow measurement and for the driving circuit for operating a sensor for flow measurement.

According to the present invention, a flow measurement device is provided. The device comprises a sensor for flow measurement. The sensor comprises a transducer arrangement with at least one first transducer component and at least one second transducer component. The at least one first transducer component and the at least one second transducer component are arranged with an at least partly common field of view in operation. The at least one first transducer component and the at least one second transducer component are configured to be operated with a phase shift.

According to the invention, the flow measurement device also comprises an operating structure for handling and positioning the transducer array. The operating structure comprises a distal end and a proximal end. The transducer is attached to the distal end of the operating structure. Further, a data connection is provided at the proximal portion, the data connection being configured to provide measured flow data.

According to the invention, the first transducer component is provided as a first ring-type structure, and the second transducer component is provided as a second ring-type structure.

According to the invention, the second transducer component is provided annular to the first transducer component.

As an effect, an ultrasound transducer with an increased opening angle, i.e. increased viewing angle or increased field of view is provided. For example, the measured volume, and thus the measured velocity profile across the vessel, in a blood flow is larger.

The increased opening angle, also referred to as acceptance angle, is suitable for large diameter vessels. While with a single transducer, due to diffraction effects, the acceptance angle may be e.g. about 25° to 30°, the increased filed of view of according to the invention provides advantages in that, like in applications having a large vessel diameter, a flow wire or other device the sensor is attached to, it is prevented that the measured flow velocity is based predominantly on measurement that includes vessel wall in the field of view or imaging path of the ultrasound transducer element and/or the blood flow velocity value measured is less dependent on the blood flow profile across the cross section of the vessel. Thus, poor signal quality results are avoided by the larger acceptance angle of the transducer.

In an example, the first transducer component and the second transducer component are arranged in a concentric manner.

According to an example, the first transducer component and the second transducer component are provided next to each other.

According to an example, the processor is configured to drive the at least one first transducer component and the at least one second transducer component of the transducer arrangement with an oscillating alternate current as operating voltage and with opposite biasing direct current voltages for achieving the phase shift; preferably, the phase shift results in an interference effect.

According to the present invention, also an ultrasound imaging system for flow measurement is provided. In an option, the system comprises a sensor for flow measurement according to one of the preceding examples. In another option, additionally or alternatively, the system comprises a flow measurement device according to one of the preceding examples. Further, an operating console is provided. The operating console is configured to operate the at least one first transducer component and at least one second transducer component of the transducer arrangement of the sensor.

According to the present invention, also a driving circuit for operating a sensor for flow measurement is provided. The driving circuit comprises a primary high voltage input for supplying an alternating electric current to at least one first ultrasound transducer element and at least one second ultrasound transducer element both acting as ultrasound transmitter. The driving circuit also comprises a secondary input for a first biasing direct current voltage and a second biasing direct current voltage. The driving circuit further comprises a common-line connecting interface with a common connection for the at least one first ultrasound transducer element and the at least one second ultrasound transducer element. The driving circuit furthermore comprises a dual-line connecting interface with a first connection for connection with the at least one first ultrasound transducer element, and a second connection for connection with the at least one second ultrasound transducer element. The driving circuit comprises a signal output for providing a signal generated by the at least one first ultrasound transducer element and the at least one second ultrasound transducer element both acting as ultrasound receiver. The primary high voltage input is switchably connectable to the at least one first ultrasound transducer element and the at least one second ultrasound transducer element. The at least one first ultrasound transducer element and the at least one second ultrasound transducer element are switchably connectable to the signal output. The first biasing voltage is supplied to the first connection, and the second biasing voltage is supplied to the second connection such that at least a phase shift is provided between the at least one first ultrasound transducer element and the at least one second ultrasound transducer element.

According to an example, the primary high voltage input comprises a first high voltage input for supplying the at least one first ultrasound transducer element via the first connection, and a second high voltage input for supplying the at least one second ultrasound transducer element via the second connection. Further, at least one of the first biasing voltage and the second biasing voltage is adjustable to adapt a degree of phase-shift between the at least one first ultrasound transducer element and the at least one second ultrasound transducer element.

According to an example, the primary high voltage input is connectable to the at least one first ultrasound transducer element and the at least one second ultrasound transducer element via the common-line. Further, the first and second biasing voltages are provided as opposite bias voltages.

According to an example, provided for a better technical understanding, a method for flow measurement is provided. The method comprises the following steps: in a first mode, it is provided a supplying at least one first transducer component and at least one second transducer component of a transducer arrangement with an alternating electric supply current. The at least one first transducer component and the at least one second transducer component are arranged with an at least partly common field of view in operation. It is further provided an operating of the at least one first transducer component and the at least one second transducer component with a phase shift by providing a first biasing direct current voltage to the at least one first transducer component and a second biasing direct current voltage to the at least one second transducer component.

Further, in an alternating manner in a second mode, it is provided the step of receiving signals from the at least one first transducer component and the at least one second transducer component.

According to an aspect, a flow measurement sensor is provided that comprises a first and a second transducer part that are operated with a phase shift to provide an enlarged viewing angle. In an example, a double-donut (or double-doughnut) structure is provided.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a sensor for flow measurement.
Fig. 2 shows a further example of a sensor with a double donut (or doughnut) structure.
Fig. 3 shows a cross-section through a sensor with a double donut structure.
Fig. 4 schematically shows an example of a flow measurement device.
Fig. 5 shows a flow measurement device in a vessel.
Fig. 6 shows an example of an ultrasound imaging system for flow measurement.
Fig. 7 indicates an acceptance angle of a standard donut shape membrane.
Fig. 8 indicates an acceptance angle of a double donut shaped membrane.
Fig. 9 shows a first example of a driving circuit to operate the two donuts in anti-phase (180° phase delay) or a selectable phase delay between 0° and 180°.
Fig. 10 indicates a second example of a driving circuit to operate the two donuts in anti-phase (180° phase delay) by operating the two donuts at opposite bias voltage polarity.
Fig. 11 shows basic steps of an example of a method for flow measurement.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

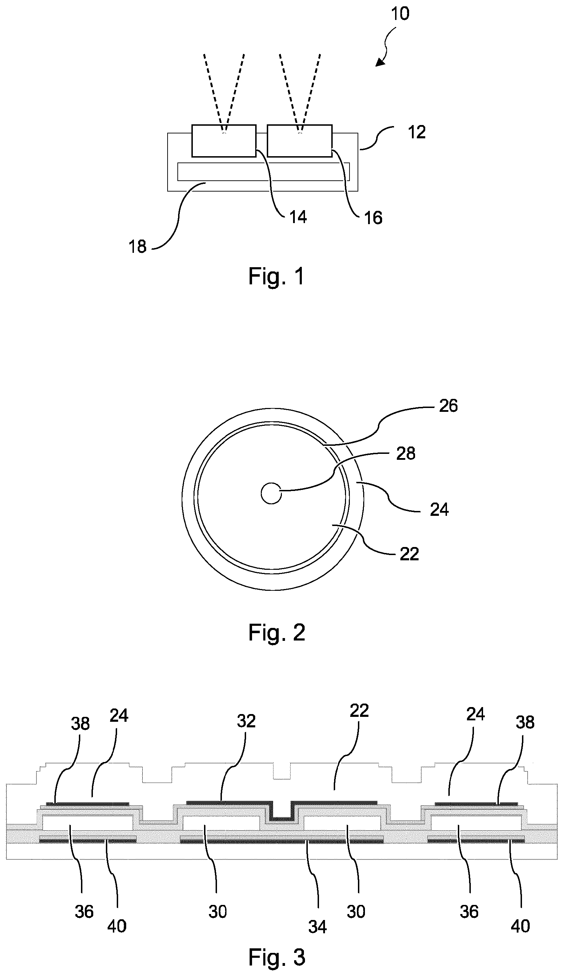

Fig. 1 schematically shows an example of a sensor 10 for flow measurement. The sensor 10 comprises a transducer arrangement 12 with at least one first transducer component 14 and at least one second transducer component 16. The at least one first transducer component 14 and the at least one second transducer component 16 are arranged with an at least partly common field of view in operation. The at least one first transducer component 14 and the at least one second transducer component 16 are configured to be operated with a phase shift.

The term "transducer component" relates to a transducer element capable of transmitting and receiving ultrasound waves.

The second transducer component is arranged annular to the first transducer component. In an option, the first transducer component 14 and the second transducer component 16 are arranged in a concentric manner. The term "concentric" relates to, for example, an arrangement with overlapping geometric centers.

The first transducer component 14 is provided as a first ring-type structure; and the second transducer component 16 is provided as a second ring-type structure. The term "ring-type" relates to circular ring forms, but also to oval, square, rectangular and polygonal ring forms.

Fig. 2 shows a further example of the sensor 10 with a double donut structure. As an example, the first and the second ring-type structures are provided as a double donut-shaped structure, wherein, in relation to the first ring-type structure, e.g. a first donut 22, the second ring-type structure, e.g. a second donut 24, has a larger outer diameter surrounding a central portion 26. The first donut may also surround a central portion 28.

In an embodiment, the first and the second ring-type structures, which are the first transducer component and the second transducer component respectively, are made of conventional piezoelectric transducers, wherein the central portion 28 and/or the annular space 26 separating the first transducer component from the second transducer component, are comprising through holes for connection of the upper or distal electrodes of the respective first and second ultrasound transducer components with electrical wires running through the elongate body of the flow measurement device 104 and which are connecting the distal electrodes to the driving and read-out electronics of the operating console 162. In an embodiment, the first transducer component is disk shaped, without the central portion 28, and it comprises only the annular space 26 separating the first transducer component from the second transducer component, which comprises a trough hole. The bottom proximal electrodes of the first transducer component and the second transducer component are connected to electrical wires running from the proximal portion of the elongate body to the proximal or bottom electrodes of the first transducer component and the second transducer component. The connection of the transducer components of the measurement device to the console may be realized through wired connection 172 or through wireless connection, in which latter case, a wireless emitter/receiver is disposed at the proximal portion of the measurement device, and which communicates wirelessly with a wireless emitter/receiver disposed within the operating console. In an alternative embodiment, the electrical connections are embedded and individually insulated within a core wire, forming the elongate body of the measurement device.

In alternative embodiments, the double donuts or the first and the second ring-type structures can be manufactured with semiconductor technology, e.g. CMOS. The transducer components may be of piezoelectric micromachined ultrasound transducers, PMUT, or capacitive micromachined ultrasound transducers, CMUT, which are exemplarily illustrated in Fig. 3. In the cross-section, the first donut 22 is arranged in the central portion, surrounded on both sides by sections of the second donut 24. The first donut 22, i.e. the first transducer, is provided with a first ring-shaped cavity 30 and with an upper electrode 32 and a lower electrode 34. The second donut 24, i.e. the second transducer, is provided with a second ring-shaped cavity 36 and with an upper electrode 38 and a lower electrode 40.

With reference to Fig. 2 and Fig. 3, the two donuts can be operated in anti-phase, i.e. a phase delay of 180° to achieve a very large acceptance angle > 60°. In addition, the acceptance angle can be tuned by selecting a phase delay between 0° and 180°.

Additionally, the implementation of the double donut concept in CMUT technology solves or reduces the problems related to a low SNR, poor bandwidth, reliability and performance issues, and resonance frequency drift and variability.

As an example, an inner donut with an outer diameter of 270 microns and an inner diameter of 35 microns is provided. The outer donut is respectively larger, for example with a diameter of 355 microns. An operation frequency of 12 MHz is provided.

As an advantage of a transducer provided as CMUT, in contrast to a transducer provided as lead zirconate titanate (PZT) transducer, there is no need of frequency tuning.

In an option, not shown in detail, the first transducer component 14 and the second transducer component 16 are provided next to each other. The term "next to each other" relates to an arrangement side by side, i.e. in an adjacent or adjoining manner.

In another option, also not shown in detail, the transducer arrangement 12 comprises a transducer array with a plurality of the first and the second ring-type structures.

In another option, also not shown in detail, the transducer arrangement 12 comprises a transducer array with a plurality of the first and second transducer components 14, 16 that are provided next to each other. The term "array" relates to a plurality of transducer elements.

In an example, shown in Fig. 1 as an option, it is also provided a processor 18 configured to drive and read out the transducer arrangement 12 with an operation frequency of preferably 12 MHz. The processor 18 provides the phase shift for operation of the at least one first transducer component 14 and the at least one second transducer component 16 of the transducer arrangement 12. For example, the processor 18 is provided as an application specific integrated circuit (ASIC).

In an example, the processor 18 is configured to drive the at least one first transducer component 14 and the at least one second transducer component 16 of the transducer arrangement 12 with an oscillating alternate current as operating voltage and with opposite biasing direct current voltages for achieving the phase shift.

In an option, the phase shift results in an interference effect.

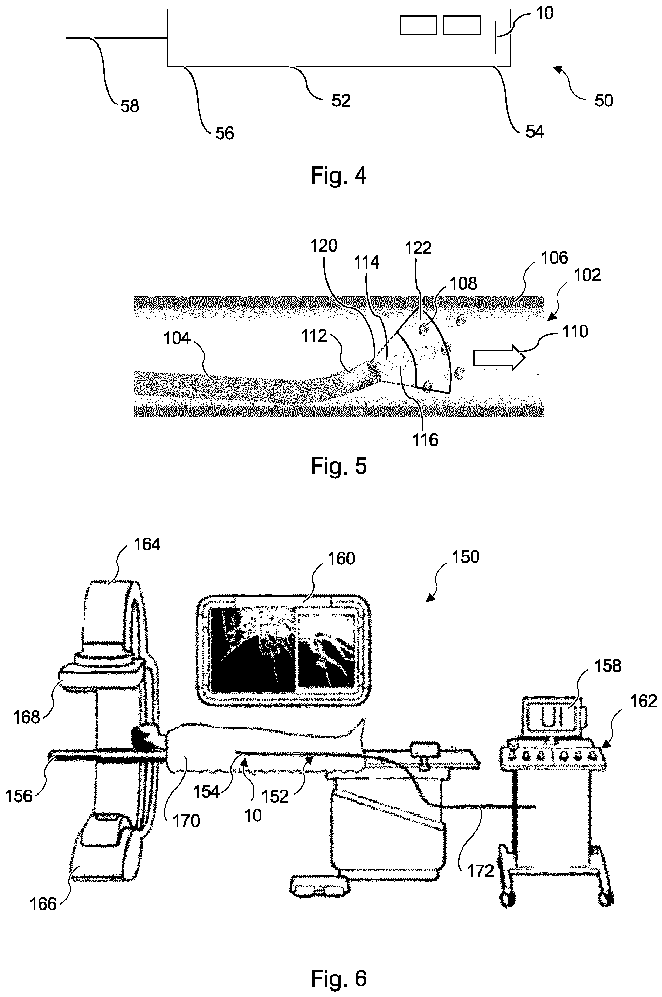

Fig. 4 schematically shows an example of a flow measurement device 50. The flow measurement device 50 comprises an example of the sensor 10 according to one of the preceding examples. Further, an operating structure 52 for handling and positioning the transducer array is provided. The operating structure 52 comprises a distal end 54 and a proximal end 56. The transducer is attached to the distal end 54 of the operating structure 52. A data connection 58 is provided at the proximal portion or the proximal end 56, the data connection 58 being configured to provide measured flow data.

The term "operating structure" relates to a physical structure that allows handling, e.g. manual handling by a user. The operating structure 52 can be a housing with portions acting as grip or handle portion. The operating structure 52 can be an elongate structure for inserting into lumen structures of a subject.

The term "distal end" relates to an end of a structure that points away from the user when operating the sensor, i.e. an end that is further away from the user and closer to the subject under examination or observation. The term "proximal end" relates to an end of a structure that points towards the user when operating the sensor, i.e. an end that is closer to the user.

For example, in case of a double donut structure, the polarity of the outer ring is reversed with respect to the inner ring by adding a 180° delay to the transmitter of the outer ring.

It is also provided as an option, to tune the acceptance angle by selecting any delay between 0° and 180°.

The operating structure 52 is an elongated structure configured for inserting at least the distal end into a lumen for intrabody vascular flow measurement. For example, an intravascular device is provided. The term "intrabody" relates to flow measurement inside a body structure, e.g. inside a vessel or lumen of an organ.

Fig. 5 schematically shows a cross section through a vessel 102 with a flow measurement device 104. The vessel 102 is indicated by vessel walls 106. Blood flow inside the vessel is indicated by blood cells 108 that move along the vessel in a blood flow direction 110. The blood flow measurement device, e.g. ultrasound device 104 comprises a transducer 112 at its distal end. The transducer 112 emits ultrasound waves 114 and receives reflected waves 116 such that ultrasound measurement data is generated and forwarded to a processor (not shown). Fig. 5 also indicates an example for a field of view 120 of the transducer with a resulting sample volume 122 in which the flow of blood is detected, i.e. measured.

In an example, the flow measurement device is a flow measurement wire.

In another example, the flow measurement device is an intravascular device.

In another example, the flow measurement device is an intraluminal device.

In another example, the flow measurement device is a guidewire with a flow measurement sensor.

The distal end is equipped with the flow sensor and is configured for inserting into a lumen, like a hollow organ or a vessel in a region of interest of a body structure, e.g. of a subject.

In another option, not shown in detail, the operating structure is an ultrasound probe configured for extracorporeal positioning for external measurement of flow inside a body lumen. The term "extracorporeal" relates to arranging the sensor outside a body structure.

Fig. 6 shows an example of an ultrasound imaging system 150 for flow measurement. The system 150 comprises an example of the device for flow measurement according to one of the preceding and following examples. Further, an interventional device 152 with an ultrasound transducer 154 attached to a distal portion of the interventional device 152 is provided. The interventional device 152 is at least data-connected to the device 10 for flow measurement. The ultrasound transducer 154 generates the plurality of ultrasound signals, which are provided to the data input (not shown in detail). As an option, Fig. 6 shows a subject support 156, e.g. a patient table.

The device for flow measurement may be provided as a movable control console or operating console 162 and may be equipped with a graphical user interface 158 like a display and/or audio user interface and further control elements. Still further, a monitor arrangement 160 is provided in the vicinity of the patient support 156, such as a ceiling- or wall-mounted display. As a further option, additional imaging systems are provided, like an X-ray imaging system 164 with an X-ray source 166 and an X-ray detector 168 mounted to a movably supported C-arm structure. An object of interest, like a subject 170, can be arranged on the subject support 156. The subject 170 is shown with cover drapes. A line 172 indicates the data-connection, which can be wired and/or wireless.

The operating console 162 is configured to operate the at least one first transducer component and at least one second transducer component of the transducer arrangement of the sensor. The term "operating console" relates to an interface provided for the user to control the operation of the sensor.

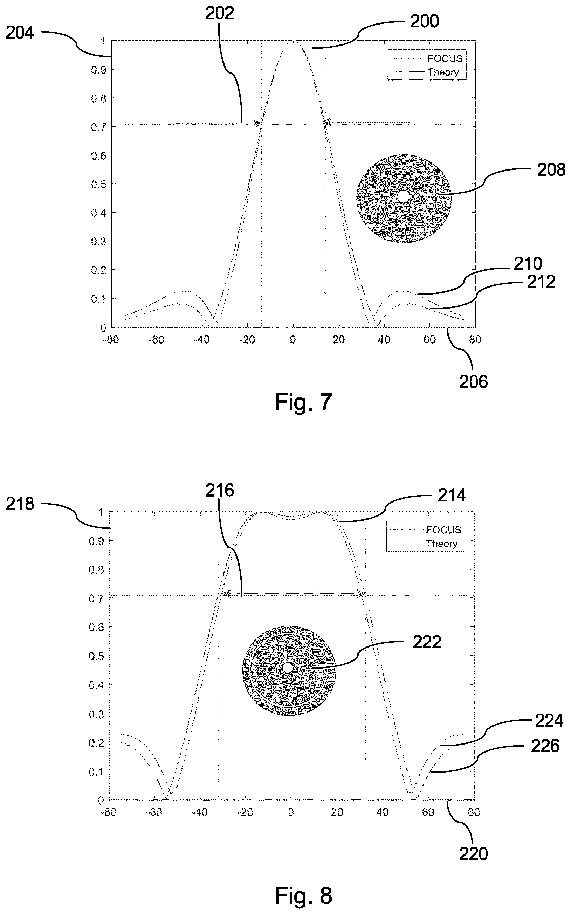

Fig. 7 indicates an acceptance angle 200 of a standard donut shape membrane, indicated by a first pair of arrows 202. A vertical axis 204 indicates e.g. normalized pressure and a horizontal axis 206 indicates the angle. A single-donut 208 is indicated as reference. A first curve 210 indicates a theoretically determined directivity function. A second curve 212 indicates a simulated directivity function.

Fig. 8 indicates an acceptance angle 214 of a double donut shape membrane, indicated by a second pair of arrows 216. A vertical axis 218 indicates e.g. normalized pressure and a horizontal axis 220 indicates the angle. A double-donut 222 is indicated as reference. A third curve 224 indicates a theoretically determined directivity function for the double-donut structure. A fourth curve 226 indicates a simulated directivity function for the double-donut structure.

As can be seen, the acceptance angle of a double donut shape is significantly larger than the acceptance angle of a single donut shape. For example, the acceptance angle of a single donut structure, for an approximately 0,7 norm pressure index, is 27°; whereas the acceptance angle of a double donut structure, for a similar approximately 0,7 norm pressure index, is 50° to 70°, e.g. 64°degrees, with a phase delay in operation of 180°.

Hence, in an example, a double donut shaped CMUT membrane is provided, see also Fig. 2 and Fig. 3. By adding an outer ring (donut) to a standard donut shaped CMUT membrane and operating the outer ring in opposite phase as the inner donut, a large acceptance angle can be achieved (see Fig. 7 and Fig. 8).

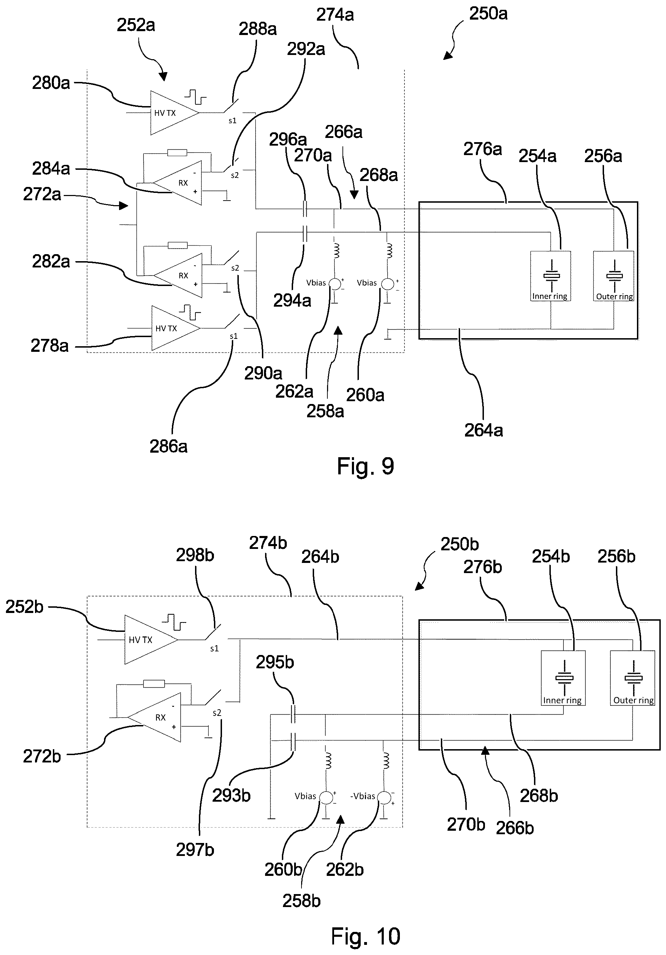

Further, as shown in Fig. 9 and Fig. 10, a driving circuit 250a and 250b for operating a sensor for flow measurement is provided. Referring to Fig. 9, the driving circuit 250a comprises a primary high voltage input 252a for supplying an alternating electric current to at least one first ultrasound transducer element 254a and at least one second ultrasound transducer element 256a both acting as ultrasound transmitter. As an example, the at least one first ultrasound transducer element 254a is provided as an inner ring of a double donut structure, and the at least one second ultrasound transducer element 256a is provided as an outer ring of a double donut structure. The driving circuit 250a comprises a secondary input 258a for a first biasing direct current voltage 260a and a second biasing direct current voltage 262a. The driving circuit 250a also comprises a common-line 264a connecting interface with a common connection for the at least one first ultrasound transducer element 254a and the at least one second ultrasound transducer element 256a. The driving circuit 250a comprises a dual-line connecting interface 266a with a first connection 268a for connection with the at least one first ultrasound transducer element 254a, and with a second connection 270a for connection with the at least one second ultrasound transducer element 256a. The driving circuit 250a comprises a signal output 272a for providing a signal generated by the at least one first ultrasound transducer element 254a and the at least one second ultrasound transducer element 256, both acting as ultrasound receiver.

In an option, the signal output 272a is split and the two output signals are processed individually.

The primary high voltage input 252a is switchably connectable to the at least one first ultrasound transducer element 254a and the at least one second ultrasound transducer element 256a. The at least one first ultrasound transducer element 254a and the at least one second ultrasound transducer element 256a are switchably connectable to the signal output 272a. The first biasing voltage 260a is supplied to the first connection 268a, and the second biasing voltage 262a is supplied to the second connection 270a such that at least a phase shift is provided between the at least one first ultrasound transducer element 254a and the at least one second ultrasound transducer element 256a.

Thus, a circuit is provided that e.g. drives the two transducer elements in anti-phase.

The term "common-line" 264a relates to a single connection supplying both transducer elements. The term "dual-line" 266a relates to two separate connections.

A first frame 274a indicates a housing structure or the like for the driving circuit 250a. A second frame 276a indicates a structure of a sensor arrangement, e.g. a structure of a flow measurement device such as an intravascular device. The driving circuit 250a can be arranged separate to the structure of the sensor arrangement. In another option, the driving circuit 250a is arranged in an integrated manner with the structure of the sensor arrangement.

The features described above are indicated with their reference number with index "a" for the option shown as example in Fig. 9, whereas similar features apply with a respective index "b" for the option shown as example in Fig. 10. Further details, in particular differing features of Fig. 9 and Fig. 10, are also described below.

In an option, the driving circuit 250a (250b) is provided for operating the sensor for flow measurement according to one of the preceding examples.

In another option, the driving circuit 250a (250b) is provided for operating the sensor in a flow measurement device according to one of the preceding examples.

In a further option, the driving circuit 250a (250b) is provided for operating the sensor in an ultrasound imaging system for flow measurement according to one of the preceding examples.

In an example, the first ultrasound transducer element 254a (254b) is provided as a first piezoelectric element, e.g. as a capacitive micromachined ultrasound transducer (CMUT) element. In an example, the second ultrasound transducer element 256a (256b) is provided as a second piezoelectric element, e.g. also as a CMUT element.

An example is provided based on CMUT ultrasound transducer using the concept of a double donut. The double donut concept allows for a large acceptance angle whereas the CMUT based ultrasound transducer allows for a large bandwidth and reduced resonance frequency variability.

The driving circuit 250a in Fig. 9 is proposed to drive the two donuts, as example. Two high voltage transmit (HV TX) signals can be chosen with a 180° phase delay to get a maximum acceptance angle. It should be noted that the acceptance angle can be tuned by choosing a phase delay between 0 and 180°.

Fig. 9 shows a first example of the driving circuit 250a to operate the two donuts in anti-phase (180° phase delay) or a selectable phase delay between 0° and 180°.

In an option, indicated in Fig. 9, the primary high voltage input 252a comprises a first high voltage input 278a for supplying the at least one first ultrasound transducer element 254a via the first connection 268a, and a second high voltage input 280a for supplying the at least one second ultrasound transducer element 256a via the second connection 270a. At least one of the first biasing voltage 260a and the second biasing voltage 262a is adjustable to adapt a degree of phase-shift between the at least one first ultrasound transducer element 254a and the at least one second ultrasound transducer element 256a.

In another option, also indicated in Fig. 9, the signal output 272a comprises a first signal output 282a for providing a signal generated by the at least one first ultrasound transducer element 254a. The signal output 272a also comprises a second signal output 284a for providing a signal generated by the at least one second ultrasound transducer element 256a.

The first high voltage input 278a is switchably connected to the first connection 268a via a first switch 286a. The second high voltage input 280a is switchably connected to the second connection 270a via a second switch 288a.

The first connection 268a is switchably connected to the first signal output 282a via a third switch 290a. The second connection 270a is switchably connected to the second signal output 284a via a fourth switch 292a.

A capacitor arrangement may be provided in the dual-line connecting interface 266a with a first capacitor 294a in the first connection 268a and a second capacitor 296a in the second connection 270a.

The common-line 264a in the example of Fig. 9 is connected to ground.

In an example, it is provided to reverse the polarity of the outer ring with respect the inner ring by reversing the bias voltage. With this concept, two acceptance angles can be selected, i.e. a narrow beam by selecting equal bias voltages or a wide beam by selecting opposite bias voltages. In an example, resonance frequencies and bandwidth can be optimized for receiving and transmitting by choosing individual optimal bias voltage levels for inner and outer ring in case of a double donut structure. As an example, the bias voltage of one of the transducers can also be chosen 0 V to 'switch' off e.g. the inner or outer element (or both).

As an alternative, the circuit in Fig. 10 is proposed to operate the two donuts, as example, in anti-phase. This is achieved by operating the two donuts at opposite bias voltage polarities. It should be noted that with this configuration, a small or large acceptance angle can be selected, i.e. by setting equal or opposite bias voltage polarities, respectively. It should also be noted that with this configuration, it is possible to slightly tune and match the resonance frequencies of the two donuts by adapting the individual bias voltage levels.

Fig. 10 indicates a second example of the driving circuit 250b to operate the two donuts in anti-phase (180° phase delay) by operating the two donuts at opposite bias voltage polarity.

In an option, indicated in Fig. 10, the primary high voltage 252b input is connectable to the at least one first ultrasound transducer element 254b and the at least one second ultrasound transducer element 256b via the common-line 264b. The first and second biasing voltages 260b, 262b are provided as opposite bias voltages.

The high voltage input 252b is switchably connected to the common-line 264b via a first further switch 298b. The common-line 264b is switchably connected to the signal output 272b via a second further switch 297b.

A capacitor arrangement may be provided in the dual-line connecting interface 266b with a first further capacitor 295b in the first connection 268b and a second further capacitor 293b in the second connection 270b.

The first connection 268b and the second connection 270b of the dual-line connecting interface 266b in Fig. 10 are connected to ground.

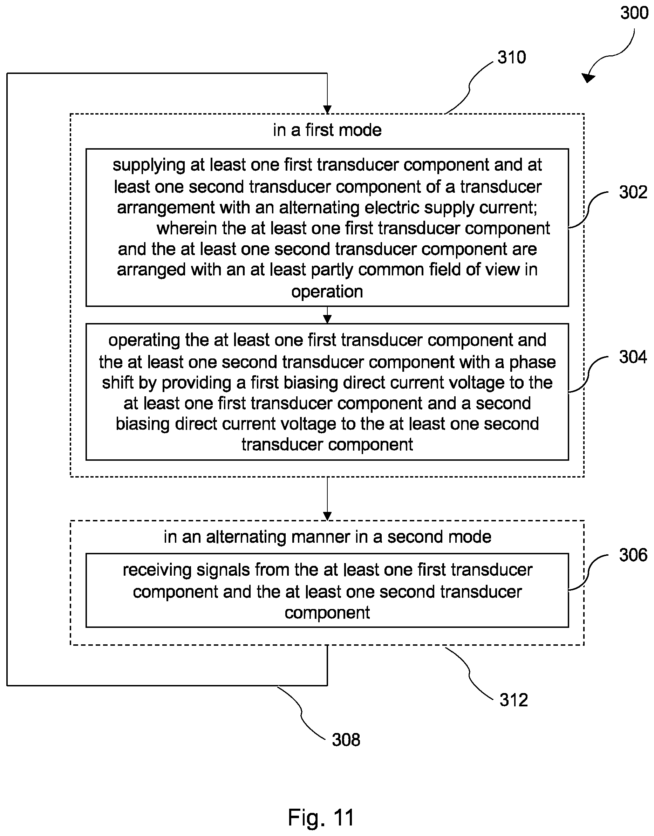

Fig. 11 shows basic steps of an example of a method 300 for flow measurement. The method comprises the following steps:
In a first mode, a supplying 302 at least one first transducer component and at least one second transducer component of a transducer arrangement with an alternating electric supply current is provided. The at least one first transducer component and the at least one second transducer component are arranged with an at least partly common field of view in operation. Further, an operating 304 of the at least one first transducer component and the at least one second transducer component with a phase shift by providing a first biasing direct current voltage to the at least one first transducer component and a second biasing direct current voltage to the at least one second transducer component is provided. The two actions, i.e. the supplying 302 with electric supply and the operating 304, are provided more or less simultaneously.

In an alternating manner in a second mode, a step of receiving 306 signals from the at least one first transducer component and the at least one second transducer component is provided.

A loop-like arrow 308 indicates an option of continuous alternating operation in the first and the second mode.

The first mode, also referred to as ultrasound sending mode, is indicated by a first frame 310. The second mode, also referred to as ultrasound receiving mode, is indicated by a second frame 312.

In an example, a computer program is provided enabling a processor to carry out the method of the preceding example.

In an example, a computer program or program element for controlling an apparatus according to one of the examples above is provided, which program or program element, when being executed by a processing unit, is adapted to perform the method steps of one of the method examples above.

In another example, a computer readable medium is provided having stored the program element of the preceding example.

The term "subject" may also be referred to as individual. The "subject" may further also be referred to as patient, although it is noted that this term does not indicate whether any illness or disease is actually present with the subject.

In another example, a computer program or a computer program element is provided that is characterized by being adapted to execute the method steps of the method according to one of the preceding examples, on an appropriate system.

The computer program element might therefore be stored on a computer unit or be distributed over more than one computer units, which might also be part of an example. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A flow measurement device (50), comprising:
a sensor (10) for flow measurement, comprising a transducer arrangement (12) with at least one first transducer component (14) and at least one second transducer component (16); wherein the at least one first transducer component and the at least one second transducer component are arranged with an at least partly common field of view in operation; wherein the at least one first transducer component and the at least one second transducer component are configured to be operated with a phase shift; and wherein the first transducer component is provided as a first ring-type structure; and the second transducer component is provided as a second ring-type structure;
wherein the second transducer component is provided annular to the first transducer component.
an operating structure (52) for handling and positioning the transducer arrangement;
wherein the operating structure is an elongated structure configured for insertion of at least the distal end into a lumen for intrabody vascular flow measurement;
wherein the transducer arrangement is attached to the distal end of the operating structure; and
wherein a data connection (58) is provided at a proximal portion of the operating structure, the data connection being configured to provide measured flow data.

2. Device according to claim 1, wherein the first transducer component and the second transducer component are arranged in a concentric manner.

3. Device according to one of the preceding claims, wherein the transducer arrangement comprises a transducer array with:
i) a plurality of the first and the second ring-type structures; or
ii) a plurality of the first and second transducer components provided next to each other.

4. Device according to one of the preceding claims, wherein a processor (18) is further provided, configured to drive and/or read out the transducer arrangement; and
wherein the processor is configured to provide the phase shift for operation of the at least one first transducer component and the at least one second transducer component of the transducer arrangement.

5. Device according to claim 4, wherein the processor is configured to drive the at least one first transducer component and the at least one second transducer component of the transducer arrangement with an oscillating alternate current as operating voltage and with opposite biasing direct current voltages for achieving the phase shift.

6. An ultrasound system (150) for flow measurement, comprising:
a flow measurement device according to one of claims 1 to 5; and
an operating console (162);
wherein the operating console is configured to operate the at least one first transducer component and at least one second transducer component of the transducer arrangement of the sensor.

7. Device according to claim 1 further comprising a Adriving circuit (250) for operating the sensor for flow measurement, the driving circuit comprising:
a primary high voltage input (252) configured for supplying an alternating electric current to at least one first ultrasound transducer component and at least one second ultrasound transducer component, in transmitting mode;
a secondary input (258) for a first biasing direct current voltage and a second biasing direct current voltage;
a common-line (264) connecting interface with a common connection for the at least one first ultrasound transducer component and the at least one second ultrasound transducer component; and
a dual-line connecting interface (266) with:
a first connection (268) for connection with the at least one first ultrasound transducer component; and
a second connection (270) for connection with the at least one second ultrasound transducer component;
a signal output (272) for providing a signal generated by the at least one first ultrasound transducer component and the at least one second ultrasound transducer component, in receiving mode;
wherein the primary high voltage input is switchably connectable to the at least one first ultrasound transducer component and the at least one second ultrasound transducer component;
wherein the at least one first ultrasound transducer component and the at least one second ultrasound transducer component are switchably connectable to the signal output; and
wherein the first biasing voltage is supplied to the first connection, and the second biasing voltage is supplied to the second connection such that at least a phase shift is provided between the at least one first ultrasound transducer component and the at least one second ultrasound transducer component.

8. Device according to claim 7, wherein the primary high voltage input comprises a first high voltage input (278a) for supplying the at least one first ultrasound transducer component via the first connection, and a second high voltage input (280a) for supplying the at least one second ultrasound transducer component via the second connection; and
wherein at least one of the first biasing voltage and the second biasing voltage is adjustable to adapt a degree of phase-shift between the at least one first ultrasound transducer component and the at least one second ultrasound transducer component.

9. Device according to claim 7 or 8, wherein the primary high voltage input is connectable to the at least one first ultrasound transducer component and the at least one second ultrasound transducer component via the common-line; and
wherein the first and second biasing voltages are provided as opposite bias voltages.

## Patentansprüche

1. Durchflussmessvorrichtung (50), umfassend:
ein Sensor (10) zur Durchflussmessung, umfassend eine Wandleranordnung (12) mit mindestens einer ersten Wandlerkomponente (14) und mindestens einer zweiten Wandlerkomponente (16); wobei die mindestens eine erste Wandlerkomponente und die mindestens eine zweite Wandlerkomponente im Betrieb mit einem mindestens teilweise gemeinsamen Sichtfeld angeordnet sind; wobei die mindestens eine erste Wandlerkomponente und die mindestens eine zweite Wandlerkomponente so ausgebildet sind, dass sie mit einer Phasenverschiebung betrieben werden; und wobei die erste Wandlerkomponente als eine erste ringartige Struktur bereitgestellt ist; und die zweite Wandlerkomponente als eine zweite ringartige Struktur bereitgestellt ist;
wobei die zweite Wandlerkomponente ringförmig um die erste Wandlerkomponente bereitgestellt ist;
eine Betriebsstruktur (52) zum Handhaben und Positionieren der Wandleranordnung;
wobei die Betriebsstruktur eine längliche Struktur ist, die ausgebildet ist, um mindestens das distale Ende in ein Lumen zur intrakorporalen Gefäßdurchflussmessung einzuführen;
wobei die Wandleranordnung am distalen Ende der Betriebsstruktur angebracht ist; und
wobei eine Datenverbindung (58) an einem proximalen Abschnitt der Betriebsstruktur bereitgestellt ist, wobei die Datenverbindung so ausgebildet ist, dass sie gemessene Durchflussdaten bereitstellt.

2. Vorrichtung nach Anspruch 1, wobei die erste Wandlerkomponente und die zweite Wandlerkomponente konzentrisch angeordnet sind.

3. Vorrichtung nach einem der vorstehenden Ansprüche, wobei die Wandleranordnung ein Wandlerarray umfasst mit:
i) einer Vielzahl von den ersten und den zweiten ringartigen Strukturen; oder
ii) einer Vielzahl von den ersten und den zweiten Wandlerkomponenten, die nebeneinander bereitgestellt sind.

4. Vorrichtung nach einem der vorstehenden Ansprüche, wobei ein Prozessor (18) weiter bereitgestellt ist, der zum Ansteuern und/oder Auslesen der Wandleranordnung ausgebildet ist; und
wobei der Prozessor so ausgebildet ist, dass er die Phasenverschiebung für den Betrieb der mindestens einen ersten Wandlerkomponente und der mindestens einen zweiten Wandlerkomponente der Wandleranordnung bereitstellt.

5. Vorrichtung nach Anspruch 4, wobei der Prozessor so ausgebildet ist, dass er die mindestens eine erste Wandlerkomponente und die mindestens eine zweite Wandlerkomponente der Wandleranordnung mit einem oszillierenden Wechselstrom als Betriebsspannung und mit entgegengesetzten Gleichstrom-Vorspannungen zur Erzielung der Phasenverschiebung ansteuert.

6. Ultraschallsystem (150) zur Durchflussmessung, umfassend:
eine Durchflussmessvorrichtung nach einem der Ansprüche 1 bis 5; und
eine Betriebskonsole (162);
wobei die Betriebskonsole so ausgebildet ist, dass sie mindestens eine erste Wandlerkomponente und mindestens eine zweite Wandlerkomponente der Wandleranordnung des Sensors betreibt.

7. Vorrichtung nach Anspruch 1, weiter umfassend eine Ansteuerschaltung (250) zum Betreiben des Sensors zur Durchflussmessung, wobei die Ansteuerschaltung umfasst:
einen primären Hochspannungseingang (252), der so ausgebildet ist, dass er mindestens eine erste Ultraschallwandlerkomponente und mindestens eine zweite Ultraschallwandlerkomponente im Übertragungsmodus mit Wechselstrom versorgt;
einen sekundären Eingang (258) für eine erste Gleichstrom-Vorspannung und eine zweite Gleichstrom-Vorspannung;
eine Verbindungsschnittstelle für eine gemeinsame Leitung (264) mit einer gemeinsamen Verbindung für die mindestens eine erste Ultraschallwandlerkomponente und die mindestens eine zweite Ultraschallwandlerkomponente; und
eine Verbindungsschnittstelle für zwei Leitungen (266) mit:
einer ersten Verbindung (268) zur Verbindung mit der mindestens einen ersten Ultraschallwandlerkomponente; und
einer zweiten Verbindung (270) zur Verbindung mit der mindestens einen zweiten Ultraschallwandlerkomponente;
einen Signalausgang (272) zur Bereitstellung eines Signals, das von der mindestens einen ersten Ultraschallwandlerkomponente und der mindestens einen zweiten Ultraschallwandlerkomponente im Empfangsmodus erzeugt wird;
wobei der primäre Hochspannungseingang umschaltbar mit der mindestens einen ersten Ultraschallwandlerkomponente und der mindestens einen zweiten Ultraschallwandlerkomponente verbindbar ist;
wobei die mindestens eine erste Ultraschallwandlerkomponente und die mindestens eine zweite Ultraschallwandlerkomponente umschaltbar mit dem Signalausgang verbindbar sind; und
wobei die erste Vorspannung an die erste Verbindung angelegt ist und die zweite Vorspannung an die zweite Verbindung angelegt ist, sodass zwischen der mindestens einen ersten Ultraschallwandlerkomponente und der mindestens einen zweiten Ultraschallwandlerkomponente mindestens eine Phasenverschiebung bereitgestellt ist.

8. Vorrichtung nach Anspruch 7, wobei der primäre Hochspannungseingang einen ersten Hochspannungseingang (278a) zur Versorgung der mindestens einen ersten Ultraschallwandlerkomponente über die erste Verbindung und einen zweiten Hochspannungseingang (280a) zur Versorgung der mindestens einen zweiten Ultraschallwandlerkomponente über die zweite Verbindung umfasst; und
wobei mindestens eine von der ersten Vorspannung und der zweiten Vorspannung einstellbar ist, um einen Grad der Phasenverschiebung zwischen der mindestens einen ersten Ultraschallwandlerkomponente und der mindestens einen zweiten Ultraschallwandlerkomponente anzupassen.

9. Vorrichtung nach Anspruch 7 oder 8, wobei der primäre Hochspannungseingang über die gemeinsame Leitung mit der mindestens einen ersten Ultraschallwandlerkomponente und der mindestens einen zweiten Ultraschallwandlerkomponente verbindbar ist; und
wobei die erste und zweite Vorspannung als entgegengesetzte Vorspannungen bereitgestellt sind.

## Revendications

1. Dispositif de mesure de débit (50), comprenant :
un capteur (10) de mesure de débit, comprenant un ensemble transducteur (12) avec au moins un premier composant de transducteur (14) et au moins un second composant de transducteur (16) ; dans lequel le au moins un premier composant de transducteur et le au moins un second composant de transducteur sont agencés avec un champ de vision au moins partiellement commun en fonctionnement ; dans lequel le au moins un premier composant de transducteur et le au moins un second composant de transducteur sont configurés pour fonctionner avec un déphasage ; et dans lequel le premier composant de transducteur est fourni comme une première structure de type annulaire et le second composant de transducteur est fourni comme une seconde structure de type annulaire ;
dans lequel le second composant de transducteur est fourni de manière annulaire par rapport au premier composant de transducteur ;
une structure de fonctionnement (52) pour la manipulation et le positionnement de l'ensemble transducteur ;
dans lequel la structure de fonctionnement est une structure allongée configurée pour l'insertion d'au moins l'extrémité distale dans une lumière pour la mesure de débit vasculaire intracorporel ;
dans lequel l'ensemble transducteur est fixé à l'extrémité distale de la structure de fonctionnement ; et
dans lequel une connexion de données (58) est fournie au niveau d'une partie proximale de la structure de fonctionnement, la connexion de données étant configurée pour fournir des données de débit mesurées.

2. Dispositif selon la revendication 1, dans lequel le premier composant de transducteur et le second composant de transducteur sont agencés de manière concentrique.

3. Dispositif selon l'une des revendications précédentes, dans lequel l'ensemble transducteur comprend un réseau de transducteurs avec :
i) une pluralité des première et seconde structures de type annulaire ; ou
ii) une pluralité des premier et second composants de transducteur fournis les uns à côté des autres.

4. Dispositif selon l'une des revendications précédentes, dans lequel un processeur (18) est en outre fourni, configuré pour piloter et/ou lire l'ensemble transducteur ; et
dans lequel le processeur est configuré pour fournir le déphasage nécessaire au fonctionnement du au moins un premier composant de transducteur et du au moins un second composant de transducteur de l'ensemble transducteur.

5. Dispositif selon la revendication 4, dans lequel le processeur est configuré pour piloter le au moins un premier composant de transducteur et le au moins un second composant de transducteur de l'ensemble transducteur avec un courant alternatif oscillant comme tension de fonctionnement et avec des tensions de courant continu de polarisation opposées pour réaliser le déphasage.

6. Système à ultrasons (150) pour la mesure de débit, comprenant :
un dispositif de mesure de débit selon l'une des revendications 1 à 5 ; et
une console de fonctionnement (162) ;
dans lequel la console de fonctionnement est configurée pour actionner le au moins un premier composant de transducteur et au moins un second composant de transducteur de l'ensemble transducteur du capteur.

7. Dispositif selon la revendication 1 comprenant en outre un circuit de commande (250) pour actionner le capteur de mesure de débit, le circuit de commande comprenant :
une entrée haute tension primaire (252) configurée pour fournir un courant électrique alternatif à au moins un premier composant de transducteur à ultrasons et à au moins un second composant de transducteur à ultrasons, en mode transmission ;
une entrée secondaire (258) pour une première tension de courant continu de polarisation et une seconde tension de courant continu de polarisation ;
une interface de connexion à ligne commune (264) avec une connexion commune pour le au moins un premier composant de transducteur à ultrasons et le au moins un second composant de transducteur à ultrasons ; et
une interface de connexion à double ligne (266) avec :
une première connexion (268) pour la connexion avec le au moins un premier composant de transducteur à ultrasons ; et
une seconde connexion (270) pour la connexion avec le au moins un second composant de transducteur à ultrasons ;
une sortie de signal (272) pour fournir un signal généré par le au moins un premier composant de transducteur à ultrasons et le au moins un second composant de transducteur à ultrasons, en mode de réception ;
dans lequel l'entrée haute tension primaire peut être connectée de manière commutable au au moins un premier composant de transducteur à ultrasons et au au moins un second composant de transducteur à ultrasons ;
dans lequel le au moins un premier composant de transducteur à ultrasons et le au moins un second composant de transducteur à ultrasons peuvent être connectés de manière commutable à la sortie de signal ; et
dans lequel la première tension de polarisation est appliquée à la première connexion, et la seconde tension de polarisation est appliquée à la seconde connexion de telle sorte qu'au moins un déphasage soit fourni entre le au moins un premier composant de transducteur à ultrasons et le au moins un second composant de transducteur à ultrasons.

8. Dispositif selon la revendication 7, dans lequel l'entrée haute tension primaire comprend une première entrée haute tension (278a) destinée à alimenter le au moins un premier composant de transducteur à ultrasons via la première connexion, et une seconde entrée haute tension (280a) destinée à alimenter le au moins un second composant de transducteur à ultrasons via la seconde connexion ; et
dans lequel au moins une de la première tension de polarisation et de la seconde tension de polarisation est réglable pour adapter un degré de déphasage entre le au moins un premier composant de transducteur à ultrasons et le au moins un second composant de transducteur à ultrasons.

9. Dispositif selon la revendication 7 ou 8, dans lequel l'entrée haute tension primaire peut être connectée au au moins un premier composant de transducteur à ultrasons et au au moins un second composant de transducteur à ultrasons via la ligne commune ; et
dans lequel les première et seconde tensions de polarisation sont fournies comme des tensions de polarisation opposées.
